(19) Europäisches Patentamt
European Patent Office
Office européen des brevets

(11) Numéro de publication: **0 627 407 A1**

(12) # DEMANDE DE BREVET EUROPEEN

(21) Numéro de dépôt: **93401375.6**

(22) Date de dépôt: **28.05.93**

(51) Int. Cl.5: **C07C 217/54**, C07C 217/62, A61K 31/135

(43) Date de publication de la demande: **07.12.94 Bulletin 94/49**

(84) Etats contractants désignés: **IT**

(71) Demandeur: **MIDY S.p.A.**
**Via Piranesi 38**
**I-20137 Milano (IT)**

(72) Inventeur: **Baroni, Marco**
**Via Carducci, 11**
**I-20010 Vanzago (IT)**
Inventeur: **Croci, Tiziano**
**Via Messina N. 55**
**I-20154 Milano (IT)**
Inventeur: **Cecchi, Roberto**
**Viale dei Tigli, 1**
**20075 Lodi, Milano (IT)**

(74) Mandataire: **Gillard, Marie-Louise et al**
**Cabinet Beau de Loménie**
**158, rue de l'Université**
**F-75340 Paris Cédex 07 (FR)**

(54) **Acide (7S)-7-[(2R)-2(3-chlorophényl)-2-hydroxyéthylamino]-5,6,7,8-tétrahydronaphtalèn-2-yloxy acétique, ses sels pharmaceutiquement acceptables, à action agoniste bêta-3 adrénergique et compositions pharmaceutiques le contenant.**

(57) La présente invention concerne à titre de nouveau composé, l'acide {(7S)-7-[(2R)-2-(3-chlorophényl)-2-hydroxyéthylamino]-5,6,7,8-tétrahydronaphtalèn-2-yloxy} acétique de formule (I)

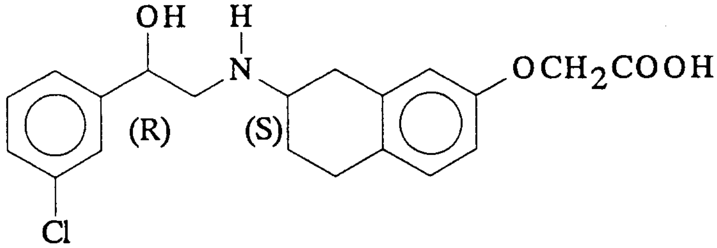

(I)

ainsi que ses sels pharmaceutiquement acceptables, à action $\beta_3$ agoniste adrénergique et les compositions pharmaceutiques le contenant.
Application : traitement et/ou prophylaxie des états pathologiques pouvant être améliorés à l'aide d'une action agoniste par médiation par les récepteurs du type $\beta_3$ adrénergique

EP 0 627 407 A1

La présente invention concerne un nouveau dérivé des phényléthanolamines ainsi que ses sels pharmaceutiquement acceptables, à action agoniste $\beta_3$ adrénergique et les compositions pharmaceutiques le contenant.

Les brevets EP 211 721 et EP 255 415 décrivent des phényléthanolamines à activité lipolytique et spasmolytique. Ces deux activités résultent d'un mécanisme d'action particulier qui a été mis en évidence seulement très récemment et qui implique les récepteurs $\beta_3$ adrénergiques.

Parmi les composés décrits dans les brevets européens sus-mentionnés, le {(7S)-7-[(2R)-2-(3-chlorophényl)-2-hydroxyéthylamino]-5,6,7,8-tétrahydronaphtalèn-2-yloxy} acétate d'éthyle, s'est montré très efficace dans la stimulation des récepteurs $\beta_3$ adrénergiques, et il a été choisi pour les essais en clinique humaine.

On a maintenent trouvé que ce composé subit des bio-modifications par les enzymes métaboliques, notamment les hydrolases, et est transformé en un métabolite, son acide correspondant, qui est aussi actif mais encore plus sélectif que le produit de départ.

On sait qu'en général l'administration du métabolite à la place de son analogue "pro-drug" est une thérapie avantageuse car le métabolite n'est plus sujet aux transformations biochimiques dans l'organisme mais, au contraire, est déjà prêt pour exercer son effet au niveau du site d'action.

De plus, la voie métabolique à laquelle le composé "pro-drug" est destiné, n'est pas unique et le médicament administré peut être métaboliquement tranformé en différents dérivés, parfois moins efficaces, parfois inactifs.

Dans le cas présent, on a trouvé de façon surprenante que le métabolite principal du composé ci-dessus est l'acide correspondant et que celui-ci est plus sélectif que le composé "pro-drug" et donc l'effet thérapeutique désiré est obtenu de façon plus sélective, les effets secondaires devenant plus faibles.

La présente invention concerne à titre de nouveau composé, l'acide {(7S)-7-[(2R)-2-(3-chlorophényl)-2-hydroxyéthylamino]-5,6,7,8-tétrahydronaphtalèn-2-yloxy} acétique de formule (I), à action $\beta_3$ agoniste adrénergique.

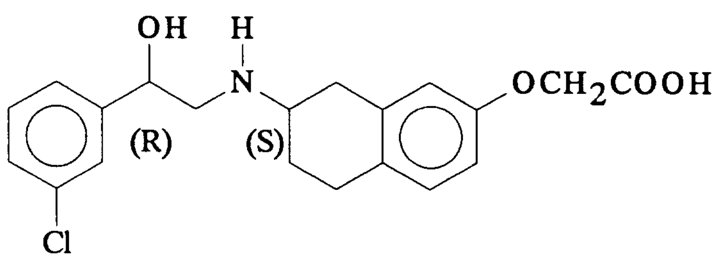

(I)

.

L'acide {(7S)-7-[(2R)-2-(3-chlorophényl)-2-hydroxyéthylamino]-5,6,7,8-tétrahydronaphtalèn-2-yloxy} acétique (I) présente deux atomes de carbone asymétrique dont la stéréochimie est fixée. Les mélanges d'isomères le contenant font également partie de la présente invention.

La présente invention concerne également les sels pharmaceutiquement acceptables du composé de formule (I).

La caractère amphotère du composé de formule (I) permet la préparation de sels pharmaceutiquement acceptables soit avec des acides pharmaceutiquement acceptables, soit avec des bases pharmaceutiquement acceptables.

Les sels avec des bases pharmaceutiquement acceptables sont par exemple ceux avec les métaux alcalins ou alcalin-terreux, tels que le sodium,le potassium, le calcium, le magnésium et ceux avec les bases organiques, telles que les amines, les acides aminés basiques (la lysine, l'arginine,l'hystidine), le trométamol, etc.

Les sels avec les acides pharmaceutiquement acceptables sont par exemple, ceux avec les acides minéraux, tels que le chlorhydrate, le bromhydrate, le borate, le phosphate, le sulfate, l'hydrogénosulfate, l'hydrogénophosphate et ceux avec les acides organiques, tels que le citrate, le benzoate, l'ascorbate, le méthylsulfate, le naphtalène-2-sulfonate, le picrate, le fumarate, le maléate, le malonate, l'oxalate, le succinate, l'acétate, le tartrate, le mésylate, le tosylate, l'α-cétoglutarate, l'α-glycérophosphate, le glucose-1-phosphate, etc.

La salification est effectuée par traitement avec l'acide ou la base choisie dans un solvent organique selon les méthodes bien connues de l'homme de métier.

Le composé de formule (I) est aisement préparé par hydrolyse de l'ester éthylique correspondant dont la méthode de synthèse est décrite dans le brevet EP 303 546.

La présente invention a également pour objet l'utilisation de l'acide de formule (I) ou de l'un de ses sels pharmaceutiquement acceptables pour la préparation de médicaments destinés au traitement et/ou à la prophylaxie des états pathologiques qui peuvent être améliorés à l'aide d'une action agoniste par médiation par les récepteurs du type $\beta_3$ adrénergique, tels que par exemple l'obésité, les affections oculaires, les troubles gastrointestinaux dûs à la contraction du muscle lisse, notamment du colon irritable.

La présente invention va maintenant être illustrée par l'exemple ci-après :

EXEMPLE 1

**Acide {(7S)-7-[(2R)-2-(3-chlorophényl)-2-hydroxyéthylamino]-5,6,7,8-tétrahydronaphtalèn-2-yloxy} acétique.**

On dissout 4,4 g (0,108 mole) de {(7S)-7-[(2R)-2-(3-chlorophényl)-2-hydroxyéthylamino]-5,6,7,8-tétrahydronaphtalen-2-yloxy} acétate d'éthyle dans 15 ml d'alcool éthylique et on y ajoute 10,5 ml de NaOH 1N. On agite pendant 30 min. à la température ambiante et 30 min. à 40-50 °C. On évapore sous pression réduite et on dissout le résidu dans 50 ml d'eau. On lave à l'éther éthylique, on sépare les deux phases et on ajoute à la phase aqueuse 10,5 ml d'acide chlorhydrique 1N. On obtient un précipité qui est trituré, filtré et lavé à l'eau pour donner 2,5 g du produit du titre. P.f. 215 °C déc.; $[\alpha]^{20}$ = -98,4(0,5% MeOH/HCl 1N).

Le composé de formule (I) ainsi que ses sels pharmaceutiquement acceptables, est un agoniste $\beta_3$ adrénergique puissant et sélectif; son activité a été évaluée à l'aide d'essais soit in vitro soit in vivo.

Pour l'évaluation de l'agonisme $\beta_3$ adrénergique sélectif, on a scumis le composé de formule (I) aux essais décrits dans le brevet EP 436 435.

Le composé de formule (I) s'est montré très actif dans l'essai in vitro du colon proximal de rat et par contre complètement inactif dans les essais sur l'utérus de rat et sur l'oreillette droite de cobaye.

De plus, on a réalisé des essais in vivo sur la motricité intestinale chez le rat anesthésié, selon la méthode décrite dans EP 255 415; le composé de formule (I) s'est aussi montré très efficace dans ce test.

L'activité agoniste du composé de formule (I), ainsi que celle de ses sels pharmaceutiquement acceptables, vis-à-vis des récepteurs $\beta_3$ adrénergiques, se manifeste par un effet stimulateur de la lipolyse et modulateur de la motricité intestinale.

Le composé de formule (I), ainsi que ses sels pharmaceutiquement acceptables, peut donc être utilisé en thérapie dans le traitement et/ou la prophylaxie des troubles gastrointestinaux chez les mammifères, tels que ceux dûs à des contractions du muscle lisse, plus particulièrement dans le colon irritable et les spasmes qui accampagnent l'ulcère peptique.

Le composé de formule (I) peut aussi être utilisé dans les cas d'obésité grâce à son effet stimulateur de la lipolyse.

On peut également envisager l'utilisation du composé de formule (I) dans le traitement des affections oculaires, notamment pour les contrôles de la pression intraoculaire et le traitement de l'hypertension oculaire et du glaucome.

La présente invention concerne également les compositions pharmaceutiques renfermant, en tant que principe actif, le composé de formule (I) ainsi que ses sels pharmaceutiquement acceptables.

Pour son utilisation thérapeutique, le produit de formule (I), pur ou en association avec toute autre substance pharmaceutiquement compatible, peut être utilisé sous des formes pharmaceutiques convenables, destinées à l'administration par voie orale, parentérale, sublinguale, rectale, transdermique et topique.

Pour l'administration orale on peut par exemple utiliser des comprimés, des dragées, des granules ou des compositions liquides, telles que des sirops, des élixirs, des émulsions, des solutions.

Pour l'administration parentérale on peut utiliser des compositions stériles injectables, aqueuses ou non aqueuses, des suppositoires pour l'administration rectale, des patches pour l'administration transdermique; le cas échéant on peut préparer des formes retard ou des formes dans lesquelles le principe actif de formule (I) est inclus dans des liposomes ou dans des cyclodextrines.

Ces formes pharmaceutiques sont préparées selon les méthodes conventionnelles, en mélangeant le principe actif de formule (I) avec les excipients et les additifs habituellement employés dans la technique pharmaceutique, tels que l'amidon, le lactose, le talc, le stéarate de magnésium, le saccharose, l'huile de paraffine, les produits mouillants, aromatisants, stabilisants, etc.

Avantageusement, les compositions pharmaceutiques selon l'invention contiennent, à titre de principe actif, de 0,01 à 500 mg du composé de formule (I) ou de l'un de ses sels pharmaceutiquement acceptables.

Le dosage approprié pour l'utilisation thérapeutique de ces compositions doit être évalué selon le cas, en considérant les caractéristiques des sujets à traiter, l'âge, le poids du corps et la gravité des affections à

traiter. En général, l'utilisation thérapeutique du composé de formule (I) prévoit l'administration d'un dosage compris entre 0,01 et 100 mg/kg par jour, de préférence entre 0,1 et 50 mg, éventuellement subdivisé en doses à administrer plusieurs fois par jour, la posologie préférée étant une à trois fois par jour.

Si l'on emploie le composé de formule (I) dans le traitement des affections oculaires, on utilise des compositions pharmaceutiques pour l'administration topique sur l'oeil, comme par exemple des suspensions, des solutions, des pommades, des gels, préparées, selon les méthodes connues, de manière appropriée pour cette application spécifique.

Les formulations ophtalmiques peuvent contenir de 0,00001 à 1% en poids du composé de formule (I), plus particulièrement de 0,0001 à 0,2 %.

La posologie suggérée pour cette thérapie topique, est comprise entre 10 ng et 1 mg de principe actif par jour, de préférence subdivisée en doses à administrer plusieurs fois par jour, la posologie préférée étant une à trois fois par jour.

**Revendications**

**1.** Acide {(7S)-7-[(2R)-2-(3-chlorophényl)-2-hydroxyéthylamino]-5,6,7,8-tétrahydronaphtalèn-2-yloxy}acétique de formule (I) ainsi ses sels pharmaceutiquement acceptables.

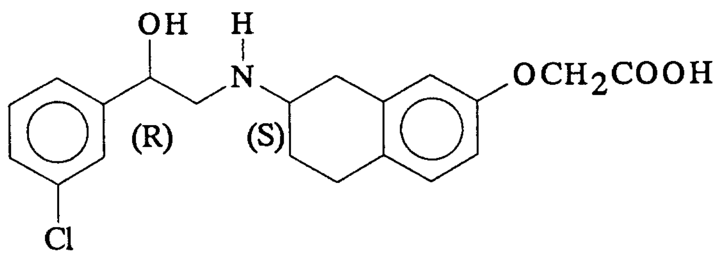

(I)

**2.** Sel de sodium du composé selon la revendication 1.

**3.** Utilisation du composé selon la revendication 1 pour la préparation d'un médicament déstinés au traitement et/ou à la prophylaxie des états pathologiques qui peuvent être améliorés à l'aide d'une action agoniste par médiation par les récepteurs du type $\beta_3$ adrénergique.

**4.** Utilisation selon la revendication 3 pour la préparation d'un médicament destiné au traitement et/ou à la prophylaxie des troubles gastrointestinaux dûs à la contraction du muscle lisse.

**5.** Utilisation selon la revendication 4 pour le traitement et/ou la prophylaxie du colon irritable.

**6.** Utilisation selon la revendication 3 pour la préparation d'un médicament destiné au traitement et/ou à la prophylaxie de l'obésité.

**7.** Utilisation de la revendication 3 pour la préparation d'un médicament destiné au traitement et/ou à la prophylaxie des affections oculaires.

**8.** Compositions pharmaceutiques contenant à titre de principe actif le composé selon la revendication 1.

**9.** Compositions pharmaceutiques selon la revendication 8 contenant de 0,01 à 500 mg de principe actif.

Office européen des brevets

# RAPPORT DE RECHERCHE EUROPEENNE

Numero de la demande

EP 93 40 1375

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y,D | EP-A-0 211 721 (SANOFI S.A., MIDY S.P.A)<br>* le document en entier * | 1-9 | C07C217/54<br>C07C217/62<br>A61K31/135 |
| Y | MANFRED E. WOLFF 'Burger's Medicinal Chemistry'<br>1980 , JOHN WILEY & SONS , NEW YORK - CHICHESTER - BRISBANE - TORONTO<br>Part I IV Edition<br>* page 178 - page 182 * | 1-9 | |
| A,D | EP-A-0 436 435 (SANOFI S.A.)<br>* le document en entier * | 1-7 | |
| A,D | EP-A-0 255 415 (SANOFI S.A., MIDY S.P.A.)<br>* le document en entier * | 1-7 | |

DOMAINES TECHNIQUES RECHERCHES (Int. Cl.5)

C07C
A61K

Le présent rapport a été établi pour toutes les revendications

| Lieu de la recherche | Date d'achèvement de la recherche | Examinateur |
|---|---|---|
| BERLIN | 09 SEPTEMBRE 1993 | RUFET J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant